# EUROPEAN PATENT APPLICATION

(11) **EP 4 772 258 A1**
(43) Date of publication of application: **08.07.2026**
(21) Application number: 24859378.2
(22) Date of filing: 06.08.2024
(51) Int. Cl.: B01D 15/00, G01N 1/10, G01N 30/00

(54) **RECOVERY METHOD, ANALYSIS METHOD, AND RECOVERY DEVICE FOR METAL IMPURITIES**

(30) Priority: 30.08.2023 JP 2023139998
(71) Applicant: Organo Corporation, Tokyo 136-8631 (JP)
(72) Inventor: MORINO, Shota, Tokyo 136-8631 (JP); KIKUCHI, Kohei, Tokyo 136-8631 (JP); SUGAWARA, Hiroshi, Tokyo 136-8631 (JP)
(74) Representative: Müller-Boré & Partner Patentanwälte PartG mbB
(86) International application number: PCT/JP2024/028116
(87) International publication number: WO 2025/047338

(57) **Abstract**

A collection method for collecting metal impurities in an organic solvent includes diluting the organic solvent with water to prepare a diluted solution, and passing the diluted solution prepared through adsorbent 12 as a sample solution to capture the metal impurities in the sample solution in adsorbent 12.

## Description

### Technical Field

The present invention relates to a collection method, an analysis method, and a collection apparatus for metal impurities.

### Background Art

Highly purified organic solvents are used in the manufacturing process of electronic devices such as semiconductors. Metal impurities contained in organic solvents can significantly affect device characteristics even in trace amounts, and therefore their concentration must be strictly controlled. For this reason, there is a need to analyze trace amounts of metal impurities in organic solvents with high accuracy.

As a method for analyzing metal impurities in organic solvents, it is generally known to introduce a sample solution sampled with a bottle directly into an analyzer such as an inductively coupled plasma mass spectrometer (ICP-MS) for analysis. On the other hand, if highly accurate analysis is required even at low concentrations of metal impurities in organic solvents that are below the detection limit of an analyzer, there is also a method in which a sample solution sampled with a bottle is concentrated by heating, and the metal impurities in the concentrated sample solution are analyzed (see, for example, Patent Literatures 1-3).

### Citation List

### Patent Literature

Patent Literature 1: JP 2001-141721 A
Patent Literature 2: JP 2002-5799 A
Patent Literature 3: WO 2020/071481 A1

### Summary of Invention

### Technical Problem

However, sampling the sample solution with the bottle not only carries the risk of contamination depending on the work environment and the proficiency of the operator, but also raises safety concerns for the operator and the surrounding environment due to liquid leakage. In addition, if the sampling site is far from where subsequent processes (concentration by heating, analysis, etc.) are performed, the bottle will need to be transported, which may be limited if the sample solution is an organic solvent. Furthermore, in the method in which the sample solution is concentrated by heating, since there is a limit to the concentration factor achievable by heating, a large amount of sample solution must be sampled to obtain sufficient sensitivity. Moreover, the sample solution is concentrated by heating in an open environment, which also increases the risk of contamination.

It is therefore an object of the present invention to collect metal impurities in an organic solvent safely and simply while preventing the occurrence of contamination.

### Solution to Problem

To achieve the above object, a metal impurity collection method of the present invention is a collection method for collecting metal impurities in an organic solvent, and includes diluting the organic solvent with water to prepare a diluted solution, and passing the diluted solution prepared through an adsorbent as a sample solution to capture the metal impurities in the sample solution by the adsorbent.

In addition, an analysis method for metal impurities of the present invention includes quantifying the metal impurities collected by the collection method described above, and calculating a concentration of the metal impurities in the organic solvent based on a result of the quantifying and a dilution factor of the diluted solution.

Furthermore, a metal impurity collection apparatus of the present invention is a collection apparatus for collecting metal impurities in an organic solvent, and includes an adsorbent for capturing the metal impurities in the organic solvent, and a sample solution passing means for diluting the organic solvent with water to prepare a diluted solution and passing the diluted solution prepared through the adsorbent as a sample solution.

According to the collection method, the analysis method, and the collection apparatus for metal impurities, the need to sample the sample solution with a bottle is eliminated, and the occurrence of various problems associated therewith can be prevented. In addition, when preparing the diluted solution, the concentration of the organic solvent can be reduced to a safe concentration (for example, a concentration that is considered a non-hazardous material under the Fire Service Act or a concentration that does not affect the human body or the environment even if leaked outside). This improves, combined with the elimination of concerns about liquid leakage, reliability in terms of safety.

### Advantageous Effects of Invention

As described above, according to the present invention, metal impurities in an organic solvent can be collected safely and simply while preventing the occurrence of contamination.

### Brief Description of the Drawings

FIG. 1 is a schematic configuration diagram of an impurity collection apparatus according to a first embodiment of the present invention; and
FIG. 2 is a schematic configuration diagram of an impurity collection apparatus according to a second embodiment of the present invention.

### Description of Embodiments

Embodiments of the present invention will be described below with reference to the drawings. Components common to the following embodiments will be denoted by the same reference numerals in the drawings, and a repetitive explanation will be omitted as appropriate. An organic solvent diluted with ultrapure water is exemplified herein as a diluted solution of the present invention, but the present invention is not limited thereto, and water having a purity lower than that of ultrapure water may be used as a diluent. A method for preparing the diluted solution by adding ultrapure water to an organic solvent is exemplified herein, but the present invention is not limited thereto, and an organic solvent may be added to ultrapure water.

### (First Embodiment)

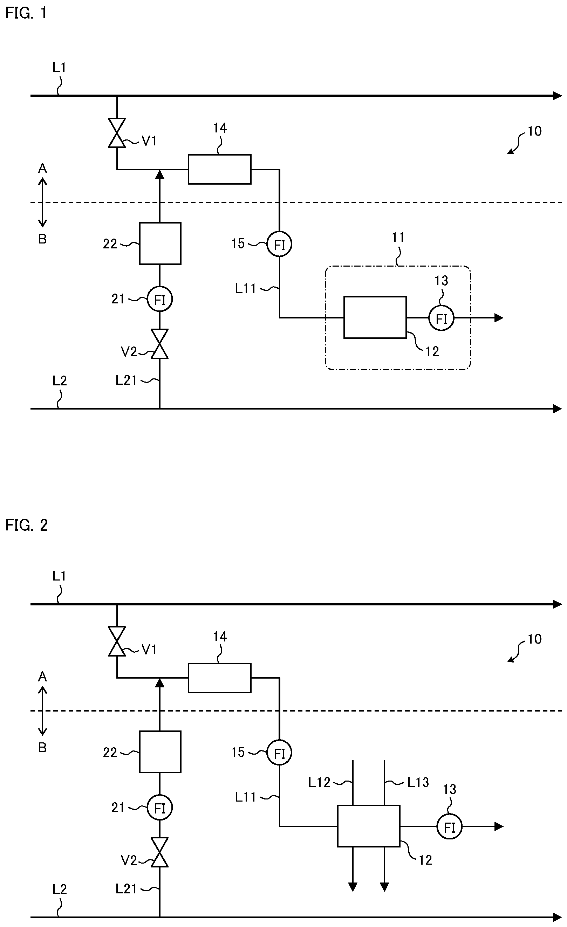

FIG. 1 is a schematic configuration diagram of an impurity collection apparatus according to a first embodiment of the present invention. It goes without saying that the illustrated configuration of the impurity collection apparatus is merely an example and may be modified as necessary, for example, by adding valves or measuring instruments.

Impurity collection apparatus 10 is intended to collect metal impurities in an organic solvent supplied through stock solution supply line L1. The target organic solvent is not particularly limited, but examples thereof include various organic solvents such as alcohols (isopropyl alcohol, methanol, ethanol, etc.), ketones (cyclohexanone, methyl isobutyl ketone, acetone, methyl ethyl ketone, etc.), alkenes (2,4-diphenyl-4-methyl-1-pentene, 2-phenyl-1-propene, etc.), esters (propylene glycol monomethyl ether acetate, isopropyl acetate, etc.), aromatics, and amines (N-methylpyrrolidone, etc.), as well as mixtures thereof.

Impurity collection apparatus 10 includes sampling line L11 and impurity collection kit 11 removably attached to sampling line L11. Sampling line L11 is connected to stock solution supply line L1 via on-off valve V1. Impurity collection kit 11 includes concentration column 12 and integrating flowmeter 13. Concentration column 12 is intended to capture and concentrate metal impurities in a sample solution supplied through sampling line L11 and is composed of a plastic container and an ion exchanger (adsorbent) packed in the container. Integrating flowmeter 13 functions to measure the total volume of sample solution passed through concentration column 12. Impurity collection kit 11 may be provided with a sealing means for sealing the interior to prevent contamination when removed from sampling line L11 and to reduce the risk of liquid leakage. Impurity collection kit 11 may also be provided with an exhaust means such as a duct or an introduction means for introducing an inert gas to prevent the interior from becoming an organic solvent vapor atmosphere.

Examples of the ion exchanger packed in concentration column 12 include an ion exchange resin, an ion adsorption membrane (i.e., a porous membrane having ion exchange capacity), and a monolithic organic porous ion exchanger; among these, a monolithic organic porous ion exchanger is preferably used. The monolithic organic porous ion exchanger is one obtained by introducing ion-exchange groups into the skeleton of a monolithic organic porous body, i.e., a porous body having numerous communication holes, serving as flow channels for a reaction solution, inside a skeleton formed of an organic polymer. Compared to typical granular ion exchange resins, the monolithic organic porous ion exchanger allows liquid to pass therethrough at a higher space velocity, thereby significantly reducing the required reaction time. In addition, the metal impurities captured thereby are easily eluted into eluents for quantitative analysis, and therefore the volume of eluent used can be reduced, so that the volume of sample solution passed therethrough can also be reduced. Furthermore, the eluent collected after passing through the monolithic organic porous ion exchanger is excellent for storage (preservation) because it is an acid-added state and the metal impurities eluted therein do not adhere to a collection container such as a bottle. In this respect as well, the monolithic organic porous ion exchanger is advantageous. As the monolithic organic porous ion exchanger, at least one of a monolithic organic porous cation exchanger and a monolithic organic porous anion exchanger may be used depending on the type of metal element to be captured. Specific examples of the monolithic organic porous ion exchanger will be described later.

Although details will be described later, in this embodiment, a diluted solution obtained by diluting an organic solvent stock solution (hereinafter also referred to simply as a "stock solution") with ultrapure water is supplied to and passed through concentration column 12 as the sample solution, rather than the stock solution itself flowing through stock solution supply line L1. As a configuration (sample solution passing means) for this purpose, impurity collection apparatus 10 includes ultrapure water addition line L21 in addition to sampling line L11 described above. Ultrapure water addition line L21 is provided to connect ultrapure water supply line L2 for passage of ultrapure water and sampling line L11 and to prepare a diluted solution by adding ultrapure water to the stock solution flowing through sampling line L11. On-off valve V2 is provided downstream of ultrapure water addition line L21.

Impurity collection apparatus 10 also includes stirring means 14 and diluted solution flowmeter 15 that are provided in sampling line L11, and ultrapure water flowmeter 21 and purification means 22 that are provided in ultrapure water addition line L21. Stirring means 14 functions to mix the stock solution with the ultrapure water added to sampling line L11. Stirring means 14 is not particularly limited but is preferably one that does not require electrical equipment because there is a risk of the organic solvent catching fire; for example, a static mixer or a long tube is used. Stirring means 14 may not necessarily be provided. Diluted solution flowmeter 15 and ultrapure water flowmeter 21 are used to calculate the dilution factor of the diluted solution, and function to measure the flow rate of the diluted solution flowing through sampling line L11 and the flow rate of the ultrapure water flowing through ultrapure water addition line L21, respectively. Purification means 22 functions to remove metal impurities from the ultrapure water to be added. Purification means 22 used may be, for example, an ion exchanger similar to that packed in concentration column 12. Purification means 22 may not necessarily be provided depending on the purity of the ultrapure water to be added. Ultrapure water addition line L21 may be provided with a check valve between ultrapure water flowmeter 21 and purification means 22.

In this embodiment, integrating flowmeter 13 is used to measure the total volume of sample solution passed through concentration column 12, but the method for measuring the total volume of sample solution passed through concentration column 12 is not limited thereto. For example, instead of integrating flowmeter 13, diluted solution flowmeter 15 may be used, so that the values measured thereby can be integrated by a separately provided computing device. If the flow rate of the diluted solution is constant, the total volume of sample solution passed through concentration column 12 can be calculated from that flow rate and the duration of passage of the sample solution through concentration column 12. Therefore, instead of integrating flowmeter 13, a flowmeter that does not require electrical equipment (e.g., a float-type flowmeter) may be provided. Alternatively, the total volume of sample solution passed through concentration column 12 can be calculated from the total volume of stock solution flowing into sampling line L11 and the total volume of ultrapure water added thereto. Therefore, a flowmeter for measuring the flow rate of the stock solution flowing into sampling line L11 may be installed in sampling line L11 upstream of the connection with ultrapure water addition line L21, so as to respectively integrate the values measured by that flowmeter and the values measured by ultrapure water flowmeter 21 by a separately provided computing device.

A method for collecting metal impurities in an organic solvent using impurity collecting apparatus 10 described above and a method for analyzing the metal impurities thus collected will here be described. The term "metal impurities", as used herein, includes not only ionic or particulate impurities but also those present in the form of compounds or complexes.

First, an organic solvent stock solution is diluted with ultrapure water to prepare a diluted solution. Specifically, on-off valve V1 on sampling line L11 is opened to supply a portion of the stock solution flowing through stock solution supply line L1 to sampling line L11, and on-off valve V2 on ultrapure water addition line L21 is opened. As a result, a portion of the ultrapure water flowing through ultrapure water supply line L2 flows into ultrapure water addition line L21, where metal impurities such as metal ions are removed by purification means 22, and is then added to the stock solution flowing through sampling line L11. The stock solution and ultrapure water are then mixed by stirring means 14 to prepare a diluted solution of the organic solvent. In this case, if necessary, the opening degree of at least one of on-off valves V1, V2 is adjusted to regulate the flow rate of the stock solution flowing into sampling line L11 and/or the flow rate of the ultrapure water flowing into ultrapure water addition line L21, thereby adjusting the dilution factor of the diluted solution. The dilution factor of the diluted solution can be calculated from the value measured by diluted solution flowmeter 15 (preferably, its moving average value) and the value measured by ultrapure water flowmeter 21 (preferably, its moving average value).

The diluted solution thus prepared is passed through concentration column 12 as a sample solution, and metal impurities in the sample solution are captured and concentrated by the ion exchanger in concentration column 12. The passage speed in this case is not particularly limited, but is preferably 2000 h⁻¹ or less, more preferably 1000 h⁻¹ or less, and particularly preferably 200 h⁻¹ or less in terms of space velocity (SV). Alternatively, it is preferably 1000 m/h or less, and more preferably 500 m/h or less in terms of linear velocity (LV). The passage duration (concentration duration) in this case depends on the type of ion exchanger packed in concentration column 12 and the speed of solution passage described above, but is not particularly limited as long as the metal impurities to be analyzed are concentrated to an extent that allows for sufficiently accurate quantification. Since the total volume of sample solution passed through concentration column 12 is required to calculate the content of metal impurities in the sample solution from the results of the quantitative analysis described below, the total volume of sample solution is measured by integrating flowmeter 13 when the sample solution is passed therethrough.

After a predetermined passage duration, on-off valve V1 on sampling line L11 and on-off valve V2 on ultrapure water addition line L21 are closed (preferably in this order) to stop the passage of the sample solution through concentration column 12. Impurity collection kit 11 is then removed from sampling line L11, preferably with the inside sealed to prevent contamination from the outside. In this case, water (ultrapure water) may be supplied from ultrapure water addition line L21 through sampling line L11, and impurity collection kit 11 may be removed after replacing the inside with the water.

Next, a replacement fluid is passed through concentration column 12 of impurity collection kit 11 that has been removed to replace the sample solution remaining in concentration column 12, particularly in the ion exchanger, with the replacement fluid. This prevents the eluent (e.g., an aqueous solution containing an acid) used in the elution treatment described below from coming into contact with the organic solvent, thereby ensuring safety. This replacement process may be performed at the site where impurity collection apparatus 10 is installed or may be performed at a location remote from that site.

The replacement fluid used is at least one of water and gas. The water used here is preferably as pure as possible, i.e., with as low a concentration of metal impurities as possible. Specifically, the metal impurity concentration is preferably reduced to less than 10 ng/L, more preferably to less than 1 ng/L, and particularly preferably to less than 0.1 ng/L. The gas used here is preferably a high-purity gas with a purity of 99.9% or higher, more preferably 99.99% or higher. The type of high-purity gas is not particularly limited, and for example, an inert gas (nitrogen, argon, helium, etc.) or oxygen may be used. Air may also be used as a high-purity gas if metal impurities have been sufficiently removed from the air.

When the sample solution is passed through the ion exchanger, the amount of metal impurities captured thereby gradually decreases along the flow direction thereof. Therefore, the flow direction of the replacement fluid is preferably the same as the flow direction of the sample solution. Thus, even if the metal impurities that have been captured by the ion exchanger are released, they can be captured again downstream thereof, thereby preventing them from being discharged from concentration column 12. When the replacement fluid used is ultrapure water, its flow speed is not particularly limited, but is preferably 20000 h⁻¹ or less, and more preferably 100 to 4000 h⁻¹ in terms of SV. Alternatively, it is preferably 1000 m/h or less, and more preferably 1 to 80 m/h in terms of LV. The duration of passage of the replacement fluid depends on the type of ion exchanger packed in concentration column 12 and the flow speed, but is not particularly limited as long as the sample solution remaining in the ion exchanger can be replaced with the replacement fluid.

Next, an eluent is passed through concentration column 12, so that the metal impurities captured by the ion exchanger in concentration column 12 are collected by being eluted into the eluent. This elution process may be performed at the site where impurity collection apparatus 10 is installed or may be performed at a location remote from that site.

The eluent used here is an aqueous solution containing an acid. The acid contained in the eluent may be either an inorganic acid or an organic acid, and examples of the inorganic acid include nitric acid, sulfuric acid, hydrochloric acid, and phosphoric acid. Among these, nitric acid, sulfuric acid, and hydrochloric acid are preferred because they facilitate the elution of ionic metal impurities and can be prepared with high purity. The acid concentration in the eluent is not particularly limited but is preferably 0.1N to 3.0N. The eluent preferably has a concentration of each metal impurity of 100 ng/L or less. Specifically, nitric acid or hydrochloric acid having a concentration of each metal impurity of 100 ng/L or less is more preferable, and nitric acid or hydrochloric acid having a concentration of each metal impurity of 10 ng/L or less is particularly preferable.

The volume of eluent passed is not particularly limited as long as the concentration of metal impurities in the eluent to be collected is higher than the detection limit of the analyzer described later, but it is preferable as small as possible, as described later. The volume of eluent passed (collected) is required to calculate the content of metal impurities in the sample solution, similarly to the total volume of sample solution passed through concentration column 12. For this purpose, for example, a method using a collection container with a measuring function or a method in which the entire volume of a previously measured amount of eluent is passed is used.

Thereafter, the metal impurities in the collected eluent are quantified, and the concentration of metal impurities in the sample solution is calculated from the obtained metal concentration. The concentration of metal impurities in the original solution is then calculated based on the calculated concentration of metal impurities and the dilution factor of the diluted solution. This quantitative analysis may be performed at the site where impurity collection apparatus 10 is installed or may be performed at a location remote from that site. Furthermore, the quantitative analysis may be performed at a location different from that of the replacement treatment and elution treatment described above.

For the quantitative determination of metal impurities, for example, an inductively coupled plasma mass spectrometer (ICP-MS), an inductively coupled plasma atomic emission spectrometer (ICP-AES), an atomic absorption spectrometer, or an ion chromatography analyzer may be used. The type of metal impurity element to be analyzed herein is not particularly limited, and for example, at least one of Li, B, Na, Mg, Al, K, Ca, Ti, V, Cr, Mn, Fe, Co, Ni, Cu, Zn, Ga, As, Sr, Mo, Ag, Cd, In, Sn, Sb, Ba, W, Au, Pt, and Pb may be analyzed. The metal impurity concentration in the sample solution can be calculated by dividing the obtained metal concentration by the concentration factor of the eluent (i.e., the value obtained by dividing the total volume of sample solution passed by the volume of eluent collected), and the metal impurity concentration in the stock solution can be calculated by dividing its calculated value by the dilution factor of the diluted solution.

According to the calculation method described above, even if the concentration of metal impurities in the stock solution is so low that it is below the detection limit of the analyzer, the concentration of metal impurities in the stock solution can be determined by increasing the concentration factor of the eluent until the concentration of the metal impurities in the eluent reaches or exceeds the detection limit. Therefore, the concentration factor of the eluent is preferably as high as possible, and thus, if the total volume of sample solution passed is the same, the volume of eluent collected is preferably as small as possible. In other words, if metal impurities can be collected with a smaller amount of eluent, even lower concentrations of metal impurities in the stock solution can be measured (evaluated) without increasing the total volume of sample solution passed. From this perspective, the flow direction of the eluent through concentration column 12 is preferably opposite to the flow direction of the sample solution therethrough. In other words, as described above, the amount of metal impurities captured by the ion exchanger gradually decreases along the flow direction of the sample solution, but by passing the eluent in the opposite direction, such metal impurities can be collected with a smaller amount of eluent.

As described above, according to this embodiment, to concentrate and collect metal impurities in a target organic solvent, a method is used in which the organic solvent is diluted with water (ultrapure water) to prepare a diluted solution, and this diluted solution is passed through concentration column 12 as a sample solution. This eliminates the need to sample the sample solution with a bottle and the operation to concentrate metal impurities in an open environment. As a result, compared to methods in which the sample solution is concentrated by heating, the risk of contamination can be reduced, and the concentration efficiency can be improved. The ion exchanger packed in concentration column 12 generally exhibits a faster ion exchange rate when in contact with water than when in contact with an organic solvent. Therefore, the speed at which the sample solution is passed through concentration column 12 can be increased, and the passage duration at this time, i.e., the time required for concentrating the metal impurities, can be shortened.

In addition, when preparing the diluted solution, the concentration of the organic solvent can be reduced to a safe concentration (for example, a concentration that is considered a non-hazardous material under the Fire Service Act, or a concentration that does not affect the human body or the environment even if leaked outside). This allows, combined with the elimination of concerns about liquid leakage when sampling with a bottle, the safe collection of metal impurities in the target organic solvent. Furthermore, since the diluted solution thus prepared is used as the sample solution, when the organic solvent is flammable, stock solution supply line L1 must be located at hazardous area A, while impurity collection kit 11 including concentration column 12 can be located at non-hazardous area B. This means that integrating flowmeter 13 of impurity collection kit 11 no longer needs to be located at hazardous area A, and diluted solution flowmeter 15 also no longer needs to be located at hazardous area A. Therefore, it is no longer necessary to use expensive explosion-proof flowmeters 13, 15, thereby increasing the cost of impurity collection apparatus 10. The term "hazardous area" as used herein refers to an area where an explosive atmosphere, generated by the leakage of flammable gases or vapors of flammable liquids into the atmosphere, exists or is likely to exist, and where the use of equipment compliant with specified explosion-proof standards is mandatory. The concentration of the organic solvent in the diluted solution is not particularly limited, but is preferably less than the concentration (predetermined value) that is subject to regulation under laws and regulations regarding hazardous materials; for example, in the case of a saturated monohydric alcohol having 1 to 3 carbon atoms, it is less than 60% by weight, which is classified as a non-hazardous material under the Fire Service Act.

Furthermore, according to this embodiment, it is also possible to avoid another problem that occurs when an organic solvent stock solution itself is passed through an ion exchanger, particularly a monolithic organic porous ion exchanger. Specifically, when an organic solvent is passed through a monolithic organic porous ion exchanger in a water-wet state, the monolithic organic porous ion exchanger may shrink (or swell); on the other hand, when water is passed through a monolithic organic porous ion exchanger that has been wetted with an organic solvent, the monolithic organic porous ion exchanger may swell (or shrink). Due to these properties, when a monolithic organic porous ion exchanger in a water-wet state is packed in a concentration column and an organic solvent is passed therethrough as a sample solution, gaps may occur between the concentration column and the monolithic organic porous ion exchanger. As a result, sufficient contact with the organic solvent may be inhibited, reducing the ability of the monolithic organic porous ion exchanger to capture metal impurities. Repeated swelling and shrinkage of the monolithic organic porous ion exchanger due to repeated use may also cause deterioration of the monolithic organic porous ion exchanger. In contrast, in this embodiment, a diluted solution obtained by diluting an organic solvent stock solution with ultrapure water is used as the sample solution, rather than the organic solvent stock solution itself. Therefore, the shrinkage of the monolithic organic porous ion exchanger can be prevented, minimizing the occurrence of the aforementioned problem.

In the illustrated embodiment, although impurity collection kit 11 including concentration column 12 and integrating flowmeter 13 is removably attached to sampling line L11, only concentration column 12 may be removably attached to sampling line L11. In other words, after passing the sample solution, only concentration column 12 may be removed from sampling line L11 (optionally after replacing the interior with water), and the remaining sample solution may be replaced with the replacement fluid.

Two types of monolithic organic porous ion exchangers will be described here as specific examples of monolithic organic porous ion exchangers that are suitably used as the adsorbent packed in concentration column 12. One is a first monolithic organic porous ion exchanger described in paragraphs [0019] to [0028] of JP 2010-234357 A, and the other is a second monolithic organic porous ion exchanger described in paragraphs [0052] to [0061] of the same. Hereinafter, the monolithic organic porous ion exchanger is also simply referred to as a "monolithic ion exchanger," and the monolithic organic porous body is also simply referred to as a "monolith." Furthermore, a monolithic organic porous intermediate, which is an intermediate (precursor) in the production of the monolith, is also simply referred to as a "monolithic intermediate."

The first monolithic ion exchanger is obtained by introducing ion-exchange groups into the monolith, and is a continuous macropore structural body in which bubble-like macropores overlap each other and these overlapping areas form apertures (mesopores) with an average diameter of 30 to 300 µm, preferably 30 to 200 µm, and particularly preferably 35 to 150 µm, in a water-wet state.

The method for producing the first monolithic ion exchanger comprises the following four steps, as described in paragraphs [0029] to [0051] of JP 2010-234357 A. (1) First, a mixture of an oil-soluble monomer without ion-exchange groups, a surfactant, and water is stirred to prepare a water-in-oil type emulsion. The water-in-oil type emulsion is then polymerized to obtain a first monolithic intermediate having a continuous macropore structure with a total pore volume of 5 to 16 ml/g. (2) A mixture is prepared which contains a vinyl monomer, a crosslinking agent having at least two vinyl groups in one molecule, an organic solvent in which the vinyl monomer and the crosslinking agent are soluble, but the polymer produced by polymerization of the vinyl monomer is not soluble, and a polymerization initiator. (3) This mixture is polymerized with being left to still stand in the presence of the first monolithic intermediate obtained in the above step (1), thereby obtaining a first monolith having a skeleton thicker than that of the first monolithic intermediate. (4) Thereafter, ion-exchange groups are introduced into the first monolith thus obtained to produce a first monolithic ion exchanger.

On the other hand, the second monolithic ion exchanger is a co-continuous structural body including a three-dimensionally continuous skeleton with a thickness of 1 to 60 µm, which is made of an aromatic vinyl polymer containing 0.3 to 5.0 mol% of crosslinked structural units among all constituent units into which ion-exchange groups have been introduced, and three-dimensionally continuous pores with a diameter of 10 to 100 µm inside the skeleton. The second monolithic ion exchanger has a total pore volume of 0.5 to 5 ml/g, an ion exchange capacity per volume in a water-wet state of 0.3 to 5 mg equivalents/ml, and ion-exchange groups uniformly distributed in the porous ion exchanger.

The second monolithic ion exchanger is produced through the following four steps, as described in paragraphs [0062] to [0083] of JP 2010-234357 A. (1) First, a mixture of an oil-soluble monomer without ion-exchange groups, a surfactant, and water is stirred to prepare a water-in-oil type emulsion. The water-in-oil emulsion is then polymerized to obtain a second monolithic intermediate having a continuous macropore structure with a total pore volume of more than 16 ml/g and 30 ml/g or less. (2) A mixture is prepared which contains an aromatic vinyl monomer, a crosslinking agent having at least two vinyl groups in one molecule and in an amount of 0.3 to 5 mol% based on the total oil-soluble monomers, an organic solvent in which the aromatic vinyl monomer and the crosslinking agent are soluble, but a polymer produced by polymerization of the aromatic vinyl monomer is not soluble, and a polymerization initiator. (3) This mixture is polymerized with being left to still stand in the presence of the second monolithic intermediate obtained in the above step (1), thereby obtaining a second monolith having a co-continuous structure. (4) Thereafter, ion-exchange groups are introduced into the second monolith thus obtained to produce a second monolithic ion exchanger.

### (Second Embodiment)

FIG. 2 is a schematic configuration diagram of an impurity collecting apparatus according to a second embodiment of the present invention. This embodiment differs from the first embodiment in that the replacement process and elution process are performed at the site where the impurity collection apparatus is installed. Such differences will be mainly described below. The various modifications described in the first embodiment are also applicable to this embodiment, and a repetitive description will be omitted.

In this embodiment, concentration column 12 is configured so that it can be supplied with a replacement fluid and an eluent; specifically, replacement fluid line L12 and eluent line L13 are connected to concentration column 12. Replacement fluid line L12 functions as a replacement fluid supply means for supplying the replacement fluid to concentration column 12 so that the sample solution remaining in concentration column 12, particularly in the ion exchanger, is replaced with the replacement fluid. Eluent line L13 functions as an eluent supply means for supplying the eluent to concentration column 12 to collect the metal impurities captured by the ion exchanger in concentration column 12 by being eluted into the eluent. Accordingly, concentration column 12 and integrating flowmeter 13 may not be detachable from sampling line L11. To measure the volume of eluent passed (collected), eluent line L13 is provided with an integrating flowmeter (not shown), or it is provided with an instantaneous flowmeter and a computing device (both not shown) that integrates the values measured thereby. Alternatively, similar to the elution process of the first embodiment, a collection container with a measuring function may be provided downstream of eluent line L13, or the entire volume of a previously measured eluent may be passed.

Thus, in this embodiment, the process from passing the sample solution through concentration column 12 to collecting the eluent can be performed continuously online at the site where impurity collection apparatus 10 is installed. Therefore, this embodiment is advantageous over the first embodiment in that since at least the replacement process is performed at the site where impurity collection apparatus 10 is installed, the need to transport concentration column 12 in which the sample solution containing the organic solvent remains is eliminated. A quantitative analyzer such as ICP-MS may be connected downstream of eluent line L13, whereby the quantitative determination of metal impurities in the eluent may be performed online. Furthermore, such a series of processes may be automated by a control means such as a controller, in which case the control means may even calculate the concentration of metal impurities in the stock solution based on the measurement results of flowmeters 13, 15, 21 and the analysis results of the quantitative analyzer.

The portion of replacement fluid line L12 downstream of concentration column 12 may be omitted so that only the supply of the replacement fluid to concentration column 12 is performed. Similarly, the portion of eluent line L13 downstream of concentration column 12 may be omitted so that only the supply of the eluent to concentration column 12 is performed. Alternatively, replacement fluid line L12 and eluent line L13 may each be connected to sampling line L11 upstream of concentration column 12. On the other hand, ultrapure water as the replacement fluid may be supplied from ultrapure water addition line L21. In either case, the replacement fluid and the eluent can be discharged through sampling line L11. Eluent line L13 may also be omitted, so that only the replacement process is performed at the site where impurity collection apparatus 10 is installed, and the elution process is performed at a location remote from that site.

### (Example)

The effects of the present invention will be described next with reference to a specific example.

In this example, a test apparatus simulating the impurity collection apparatus shown in FIG. 2 was used to collect and analyze metal impurities in an organic solvent, and the collection rate of each metal element was calculated.

Specifically, first, a diluted solution obtained by diluting an organic solvent stock solution with ultrapure water was passed through a concentration column as a sample solution, and the metal elements contained in the sample solution were captured and concentrated by the ion exchanger in the concentration column. A monolithic organic porous cation exchanger was used as the ion exchanger packed in the concentration column. Isopropyl alcohol (IPA) adjusted so that the concentration of each of the metal elements, Li, Na, Mg, Al, K, Ca, V, Mn, Fe, Co, Ni, Cu, Zn, Ga, Sr, Ag, Cd, In, Ba, and Pb, were 100 ng/L was used as the organic solvent stock solution, and it was diluted 2-fold with ultrapure water. In other words, the opening of each on-off valve was adjusted so that the values measured by the diluted solution flowmeter and ultrapure water flowmeter were 10 mL/min and 5 mL/min, respectively. Then, 600 mL of the diluted solution was passed through the concentration column at an SV of 150 h⁻¹. A long tube was used as the stirring means, but no purification means is provided in the test apparatus in this example.

Next, as a replacement fluid, 50 mL of ultrapure water was passed through the concentration column at an SV of 150 h⁻¹, and then high-purity nitrogen gas was supplied at a flow rate of 4 L/min to replace the sample solution remaining in the concentration column with the replacement fluid. The supply of replacement fluid to the concentration column was performed using the sampling line in both cases.

Next, an eluent was passed through the concentration column through the sampling line, so that the metal elements in the sample solution captured by the ion exchanger in the concentration column were collected by being eluted into the eluent. 2N nitric acid was used as the eluent, and 50 mL thereof was passed through the column at an SV of 150 h⁻¹. Thereafter, the content of each metal element (amount of each metal) in the collected eluate was measured using ICP-MS, and the obtained metal concentration was divided by the concentration factor of the eluent (in this example, 600 mL/50 mL = 12 times) to calculate the metal concentration in the sample solution. Furthermore, the calculated metal concentration was divided by the dilution factor of the diluted solution (2 times in this example) to calculate the metal concentration in the stock solution. The metal concentration in the stock solution was separately measured, and the collection rate, which is the ratio (percentage) of the above calculated value to the measured value, was calculated. Table 1 shows the calculation results of the collection rate of each metal element in the stock solution.

| | Li | Na | Mg | Al | K | Ca | V | Mn | Fe | Co |
|---|---|---|---|---|---|---|---|---|---|---|
| Collection rate [%] | 95 | 105 | 93 | 89 | 100 | 135 | 78 | 87 | 89 | 89 |
| | | | | | | | | | | |

| | Ni | Cu | Zn | Ga | Sr | Ag | Cd | In | Ba | Pb |
|---|---|---|---|---|---|---|---|---|---|---|
| Collection rate [%] | 96 | 83 | 99 | 81 | 86 | 92 | 91 | 71 | 87 | 88 |

Referring to Table 1, it can be seen that, for all of the metal elements analyzed, the collection rate falls within the range of 100±35%. Therefore, it was confirmed that even when a diluted solution obtained by diluting an organic solvent stock solution with ultrapure water is used as the sample solution, rather than the organic solvent stock solution itself, metal impurities can be collected with a high collection rate.

### Reference Signs List

- 10: Impurity collection apparatus
- 11: Impurity collection kit
- 12: Concentration column
- 13: Integrating flowmeter
- 14: Stirring means
- 15: Diluted solution flowmeter
- 21: Ultrapure water flowmeter
- 22: Purification means
- L1: Stock solution supply line
- L2: Ultrapure water supply line
- L11: Sampling line
- L12: Replacement fluid supply line
- L13: Eluent supply line
- L21: Ultrapure water addition line
- A: Hazardous area
- B: Non-hazardous area

## Claims

1. A collection method for collecting metal impurities in an organic solvent, comprising:
diluting the organic solvent with water to prepare a diluted solution; and
passing the diluted solution prepared through an adsorbent as a sample solution to capture the metal impurities in the sample solution by the adsorbent.

2. The collection method of claim 1, further comprising:
passing at least one of water and gas as a replacement fluid through the adsorbent through which the sample solution has been passed, so that the sample solution remaining in the adsorbent is replaced with the replacement fluid; and
passing an eluent through the adsorbent through which the replacement fluid has been passed, so that the metal impurities captured by the adsorbent are collected by being eluted into the eluent.

3. The collection method of claim 1 or 2, wherein the preparing comprises diluting the organic solvent with water so that a concentration of the organic solvent in the diluted solution is less than a predetermined value.

4. The collection method of claim 1 or 2, wherein the adsorbent is a monolithic organic porous ion exchanger.

5. The collection method of claim 1 or 2, wherein the organic solvent is an alcohol.

6. An analysis method for metal impurities, comprising:
quantifying the metal impurities collected by the collection method of claim 1 or 2; and
calculating a concentration of the metal impurities in the organic solvent based on a result of the quantifying and a dilution factor of the diluted solution.

7. The analysis method of claim 6, wherein the preparing, the capturing, and the quantifying are performed online continuously.

8. A collection apparatus for collecting metal impurities in an organic solvent, comprising:
an adsorbent for capturing the metal impurities in the organic solvent; and
a sample solution passing means for diluting the organic solvent with water to prepare a diluted solution and passing the diluted solution prepared through the adsorbent as a sample solution.

9. The collection apparatus of claim 8, further comprising:
a replacement fluid supply means for supplying at least one of water and gas as a replacement fluid to the adsorbent; and
an eluent supply means for supplying an eluent to the adsorbent.

10. The collection apparatus of claim 8 or 9, wherein the adsorbent is a monolithic organic porous ion exchanger.
